# EUROPEAN PATENT APPLICATION

(11) **EP 0 648 844 A2**
(43) Date of publication of application: **19.04.1995**
(21) Application number: 94114625.0
(22) Date of filing: 16.09.1994
(51) Int. Cl.: C12Q 1/68

(54) **Method of primary screening of carriers having abnormal genetic base sequences**

(30) Priority: 20.09.1993 JP 256497/93
(71) Applicant: HITACHI ELECTRONICS ENGINEERING CO., LTD., Chiyoda-ku, Tokyo 100 (JP)
(72) Inventor: Sakaki, Yoshiyuki, Yokohama, Kanagawa-ken 236 (JP); Hattori, Masahira, Fuchu-shi, Tokyo 183 (JP); Hagiwara, Hisashi, Fuchu-shi, Tokoy 183 (JP)
(74) Representative: Strehl Schübel-Hopf Groening & Partner

(57) **Abstract**

Human extracted DNA is treated by PCR and a sequence technique. Fluorophore-labelled DNA fragments are subjected to gel electrophoresis and illuminated with laser light. The emitted fluorescence is detected and the output waveform is compared with the reference waveform of a normal subject to check for the presence of any abnormal site in the base sequence of interest. The labelled DNA fragments have sizes of no more than 500 base pairs and they are electrophoresed over tracks not longer than 100 mm. The method is suitable for primary screening and only carriers having abnormal base sequences in their genes can be located from a large population of subjects with great rapidity and at low cost.

## Description

### BACKGROUND OF THE INVENTION

This invention relates to the analysis of DNA base sequences. More particularly, the invention relates to a method of primary screening for detecting human hereditary diseases by analyzing the base sequences of human DNA.

With the recent advances in genetic engineering, it has been found that many human hereditary diseases originate from mutations in the base sequences of DNA and other proteins. In addition, the progress in the mapping of base sequences has urged researchers to conduct gene therapy on some diseases by utilizing the extracorporeal cell culture through selective cultivation of normal cells so that cells having an abnormal base sequence are brought to the normal condition and then putting the so normalized cells back into the living body.

Under the circumstances, a number of techniques have been developed for detecting variations in the sites of base substitutions and they all require a final verification to be done by performing highly reliable base sequence analyses. Since those techniques are only capable of indirect identification of variations by comparing the sizes of DNA fragments, their results must be rechecked by base sequence analyses that are capable of direct detection of the variations.

Diagnoses of hereditary diseases in fetus have heretofore been conducted by quantitative determination of abnormal metabolites, by enzymological approaches and the like and whichever method is to be used, the expression of characters has been absolutely necessary for the success of fetal diagnoses. In contrast, the DNA diagnostic approach does not require the expression of characters in the cells of amniotic fluid or placental villi as long as cellular, villous, blood and other specimens are available. Because of this advantage, the DNA-based approach is capable of diagnosing phenylketonuria (PKU), ornithine transcarbamylase (OTC) deficiencies and other diseases that have defied the fetal diagnostic approach.

A major reason for the recent advances in the DNA diagnoses of hereditary diseases is the wide-spread use of polymerase chain reaction (PCR). The PCR has the advantages of requiring only small amounts of samples to be analyzed and assuring highly operational convenience; in addition, the adoption of PCR has enabled the intended results to be attained within comparatively short periods of time.

The currently employed practical DNA diagnoses including the step of PCR are classified into the following five basic processes:
(1) hybridization of the PCR product with an allele-specific oligonucleotide (ASO) probe;
(2) electrophoresis of the PCR product;
(3) digesting the PCR product with a restriction enzyme, followed by electrophoresis;
(4) determining the base sequence of DNA in the PCR product; and
(5) digesting the PCR product with a restriction enzyme, followed by polymorphic analysis through electrophoresis and hybridization.

The first process relying upon the hybridization of the PCR product with an ASO probe is the most extensively used today to detect variations in DNA base sequences since it is applicable to practically all gene variations if the base sequences of the normal and variant genes are known. Consider, for example, the case of phenylketonuria (PKU). This hereditary disease is caused by a mutation in which arginine that is the amino acid at position 408 in the gene of phenylalanine hydroxylase (PHA) changes to tryptophan as a result of the change of the triplet code CGG to TGG. Detection of this mutation starts with preparing PCR primers 1 and 2 in such a way that they hold the mutation therebetween and then DNA amplification is conducted, with primer 1 being attached to a portion complementary to residues 2 - 21 of exon 12 and with a mismatched C residue being attached to the 3' end so as to introduce a new site for restriction enzyme. This C residue is located in a position just next to the site of mutation toward the 5' end so that if the sequence is normal, it is digestible with restriction enzyme MspI whereas it is not digestible with MspI but digestible with restriction enzyme BstNI if the sequence is abnormal. Primer 2 is a 19mer that is complementary to the sequence of a site that is distant from the site of mutation by about 170 bases toward the 3' end. If amplification is done by PCR with the set of primers 1 and 2 and if the product is separated by agarose gel electrophoresis, the amplified product of 167 base pairs (bp) is verified. If the sequence is normal, the PCR product can be digested with MspI to separate into two fragments, one having a length of 21 bp and the other 146 bp; however, the same PCR product cannot be cleaved with BstNI in the absence of a relevant recognition sequence. Therefore, three samples, the first being digestible with neither restriction enzyme, the second digested with MspI, and the third digested with BstNI, can be separated by agarose gel electrophoresis and the separated double-stranded DNA in the gel is stained with ethidium bromide and verified by illumination with ultraviolet radiations. The DNAs in the first and third samples are separated at a position of 167 bp whereas the separated product of the second sample can be verified in two positions, one corresponding to 146 bp and the other to 21 bp. The same result is obtained when analyzing the DNA of a patient with PKU: the DNAs in the first and second samples are separated at a position of 167 bp whereas the DNA from the third sample is separated at two positions, one corresponding to 146 bp and the other to 21 bp. Thus, one can verify the occurrence of a base substitution.

A problem with the first process is that its applicability is limited by the combination of the base sequence at the site of substitution with the recognition sequence of a restriction enzyme and it has no use unless a convenient combination is adopted.

It is an established fact that hepatitis B virus (HBV) is infected by various routes including blood transfusion. In view of the frequent shifting from hepatitis B to hyperacute hepatitis, HBV requires a particularly correct diagnosis. Viral detection has heretofore been conducted by methods that rely upon an immune reaction targeting the Hbe antigen. However, in many cases did hepatitis B turn to hyperacute hepatitis although its viral content was found to be insufficient as a result of the antigen-antibody reaction and this has remained a mystery. Recently, it was discovered that in HBV, the 28th triplet code in the pre-C region had experienced a mutation from TGG (tryptophan) to the termination codon TAG and this had led to the finding that dysfunction of the gene in that region was the reason for the failure to produce the Hbe antigen. Therefore, even if amplification by PCR is performed with primers prepared in such a way that they hold the region of interest therebetween, it is unable to tell whether the virus at issue is of a wild or a variant type and no reliable diagnosis can be effected unless the base sequence of the virus is determined.

The numbers of people who are suspected of suffering from hereditary diseases such as PKU and those who are potential carriers of HBV are great many. If such carriers who are presently normal but who have a high risk for the manifestation of the disease in the future can be spotted and given an appropriate treatment, they can be prevented from becoming victims to the diseases. However, it is not economical to conduct the above-described precise diagnosis on all suspects. Intensive efforts have so far been made to develop methods capable of precise diagnosis of various diseases but no convenient method has yet been developed that is capable of distinguishing carriers having gene mutations from normal non-carriers.

### SUMMARY OF THE INVENTION

An object, therefore, of the invention is to provide a convenient method of primary screening for detecting carriers having gene mutations.

To attain this object, the present invention provides a method of primary screening for carriers having an abnormal DNA base sequence that comprises the steps of amplifying a human extracted DNA by PCR, treating the PCR amplified product by a sequence technique to prepare DNA fragments, provided that the DNA is labelled with a fluorophore during the treatment by said sequence technique, then injecting the fluorophore-labelled DNA fragments into dents at the upper end of the electrolyte layer in the electrophoresis plate of a gel electrophoretic apparatus, applying a voltage to said electrolyte layer so that said DNA fragments are migrated, applying laser light to the migrating DNA fragments in a position a predetermined distance away from said dents, receiving the emitted fluorescence from said fragments by fluorescence detecting means, and outputting the reception signal as a waveform, characterized in that the fluorophore-labelled DNA fragments prepared by said sequence technique have lengths not more than 500 base pairs, that the length of electrophoresis tracks extending from the bottom of said dents to the point of incidence of laser light is no more than 100 mm, and that the base sequence of the DNA in each fraction is checked for the site of mutation by comparing said waveform output with the reference waveform of a normal subject.

Thus, according to the method of the invention, samples of DNA fragments as prepared by PCR are labelled with a suitable fluorophore and electrophoresed to yield separate nucleotides, which are illuminated with laser light to emit fluorescence. The sequences of the individual DNA fragments are determined from the detected waveforms of the emitted fluorescence. Comparing the base sequence of a DNA fragment from a normal subject with the base sequence of a carrier having an abnormal gene, the operator notes one or more mismatches between the two sequences. By checking for any such sequence mismatch, the operator can rapidly locate only carriers from a large number of subjects. A precise examination need only to be conducted on the carriers who have been found to harbor abnormal base sequences and appropriate diagnosis and/or treatment may subsequently be done. Thus, compared to the case of performing a precise examination on all subjects, the overall time and cost for examinations are drastically reduced by applying the primary screening method of the invention.

### BRIEF DESCRIPTION OF THE DRAWINGS

Fig. 1 is a flowchart of the primary screening method of the invention;
Fig. 2 shows conceptually the scheme of performing gel electrophoresis on fluorophore-labelled DNA fragments in the primary screening method of the invention;
Fig. 3 shows the waveforms of output signals for fluorophore-labelled DNA fragments as produced in a period of 15 - 25 minutes in Example 1;
Fig. 4 shows the waveforms of output signals for fluorophore-labelled DNA fragments as produced in a period of 20 - 35 minutes in Example 1;
Fig. 5 shows the waveforms of output signals for fluorophore-labelled DNA fragments as produced in a period of 30 - 40 minutes in Example 1;
Fig. 6 shows the waveforms of output signals for fluorophore-labelled DNA fragments as produced in a period of 40 - 50 minutes in Example 1; and
Fig. 7 shows the waveforms of output signals for fluorophore-labelled DNA fragments as produced in a period of 50 - 58 minutes in Example 1.

### THE PREFERRED EMBODIMENTS OF THE INVENTION

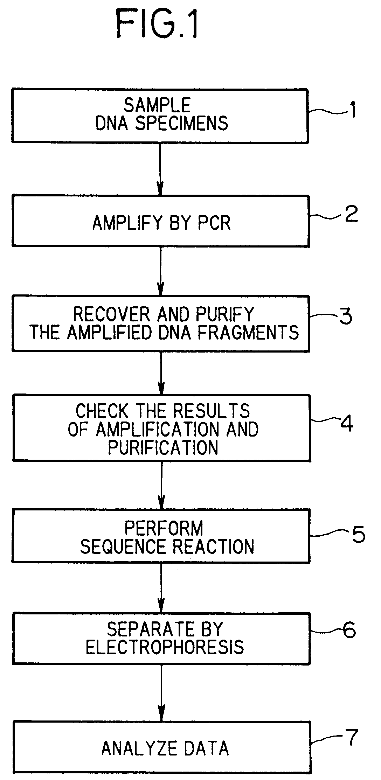

Fig. 1 is a flowchart of the primary screening method of the invention. The method starts with sampling DNA specimens from the subjects under primary screening (block 1). The DNA specimens may be obtained from any suitable origins including blood, cells, villi, lymphocytes, etc. Blood is preferred from the viewpoint of ease in sampling. The sampled DNA specimens are then amplified by PCR (block 2). Amplification by PCR can be performed automatically with any commercial PCR apparatus. Further details of amplification by PCR will be given later in this specification. The DNA fragments as amplified by PCR are recovered and purified (block 3). The amplified DNA fragments can be recovered by any suitable methods such as the polyethylene glycol (PEG) precipitation technique, the glass powder method and the gel column procedure. The PEG precipitation technique is preferred since it enables the amplified DNA fragments to be recovered and purified simultaneously. Purification of the recovered DNA fragments can be implemented by any suitable method such as dissolving them in physiological brine. Subsequently, check is made to know whether the amplification and purification have been accomplished successfully (block 4). To this end, a mini-electrophoresis apparatus is used with an agarose gel electrolyte layer and the recovered DNA fragments are electrophoresed to determine whether: (i) only the single DNA species that is required has been amplified normally; (ii) other DNA species have also been amplified (probably due to contamination); and (iii) the required DNA species is observed but in a limited quantity (probably due to weak bands or loss during purification). In the second and third cases, amplification by PCR must be repeated. However, the method of the invention is intended to perform primary screening for an abnormal genetic base sequence and may, if desired, be implemented without the step of "checking the results of amplification and purification". The recovered and purified DNA fragments are then subjected to a sequence reaction (block 5). The sequence reaction can also be performed automatically with any commercial PCR apparatus. Further details of the sequence reaction are also given later in this specification. After the end of the sequence reaction, the individual DNA fragments are electrophoresed with an automated DNA sequencer (block 6). Finally, the electrophoretic data are analyzed to pick up only those subjects who are suspected of having abnormal genetic base sequences (block 7).

For details of the operating principle of PCR, reference may be had to "Jikken Igaku (Experimental Medicine)", vol. 7, No. 2, pp. 14-18 (1989). The template DNA to be used in the sequence reaction when implementing the primary screening method of the invention is prepared by any known PCR technique in the following manner. First, primers for amplification by PCR (e.g. oligonucleotides) are designed in such a way that they hold therebetween the sites already known for base substitution. Preferably, the primers are set at sites that are at least 30 bp distant from the sites of substitution. The region to be amplified is preferably of such a size that its length is 100 - 500 bp holding the site of substitution therebetween. The primers themselves are at the "favorable sites of sequence" that satisfy the conditions set forth above and they should preferably be designed by selecting lengths of about 18 - 30 bp. The term "favorable sites of sequence" as used herein means the following: (1) they do not contain any specific sequences such as Alu and Ll that occur in human base sequences at intervals of several hundred base pairs; (2) the two primers should be located at such sites of sequence that their melting temperatures (Tm) are preferably identical to each other because different Tm values will introduce difficulty in pairing; (3) the sites are designed in positions where the numbers of adenines (A), thymines (T), guanines (G) and cytosines (C) contained have been adjusted in such a way that the calculated Tm value will be about 60°C although the exact value depends on the specificity of the sequence; (4) the base at the 3' end of the sequence of each primer is G or C; and (5) the eight bases at the 3' end of the sequence of each primer contains more G and C than A and T. The process of amplification by PCR consists basically of the repetition of dissociation, pairing and extension and the basic conditions for the respective steps are set as follows: 94°C x 60 sec (dissociation); 56°C x 60 sec (pairing); and 72°C x 60 sec (extension). Depending on the size of amplification (i.e. the number of base pairs) and the conditions for the sequences of the primers, the pairing temperature time, the extension time and other parameters may be varied to search for the optimal conditions for the region to be amplified.

The sequence reaction is performed using fluorophore-labelled sequencing primers. Labelling the primers with a fluorophore can be easily implemented using a commercial DNA synthesizer. The fluorophore is bound to the 5' end of the primers. The fluorophores that can be used in the method of the invention are not limited to any particular type. If laser light is used as an excitation light source, a suitable fluorophore can be determined in consideration of the combination of the excitation wavelength of the laser light with the emission wavelength of the fluorophore. Exemplary fluorophores that can be used in the invention include fluorescein isothiocyanate (FITC), eosine isothiocyanate (EITC), tetramethyl rhodamine isothiocyanate (TMRITC), substituted rhodamine isothiocyanate (XRITC) and sulforhodamine 101 acid chloride (commercially available under the trademark "TEXAS RED"). If a He-Ne laser operating at a wavelength of 594 nm is to be used as an excitation light source, TEXAS RED having a maximum excitation wavelength of 596 nm and a maximum emission wavelength of 615 nm is preferably used as the fluorophore. If an argon ion laser operating at a wavelength of 488 nm is to be used as an excitation light source, FITC is preferably used as the fluorophore. Chemoluminescent reagents may also be used if they do not interfere with the hybridization of primers with the DNA chains (PCR product) or the reaction of extension toward the 3' terminal end by polymerase.

The sequencing primer is preferably designed in an appropriate position that takes the following conditions into account:
(1) the primer's size should be 20 - 25 bp;
(2) it should be located inside the primer that has been used to prepare the template DNA chains (within the amplified DNA chains); in other words, a separate new primer should be bound inside one of the two existing primers;
(3) it should be designed in a position at least 20 bp apart from the site of substitution and in a direction that permits searching for the site of substitution;
(4) it should not contain any specific sequences such as Alu and Ll that occur in human base sequences at intervals of several hundred base pairs; and
(5) it should be designed in positions where the numbers of adenines (A), thymines (T), guanines (G) and cytosines (C) contained have been adjusted in such a way that the calculated Tm value will be about 70°C although the exact value depends on the specificity of the sequence.

Using the sequencing primer satisfying these conditions, the DNA polymerase reaction (i.e., sequence reaction) is carried out to prepare DNA fragments. The sequence reaction is performed by cycle sequencing utilizing a heat-resistant DNA polymerase (e.g. Taq or Vent enzyme). The termination reaction is performed by the Sanger method. Consider, for example, the case of preparing a group of DNA fragments terminated with adenine and which are to be used for adenine (A) detection. If dATP is incorporated at the terminal end of the DNA polymerase subsequent to the primer, the synthesis is continued to have the DNA chain extended; if ddATP is incorporated, the synthesis will stop. Therefore, by varying the timing of incorporating ddATP, one can prepare DNA fragments having a desired number of base pairs (or bp number). When implementing the method of the invention, the sequence reaction is carried out in such a way that the bp number of DNA fragments will be 500 or less, preferably 400 or less, most preferably 300 or less. The lower limit of the bp number of DNA fragments will in no way assume any particular value but it is preferably about 100 in consideration of the bp number of the primer. If the bp number of DNA fragments exceeds 500, an unduly long time will be spent in electrophoretic separation, which is definitely unfavorable for the purpose of primary screening. If the bp number of DNA fragments is about 300, electrophoretic separation will end in about one hour, which is short enough to justify the use of the invention method in primary screening.

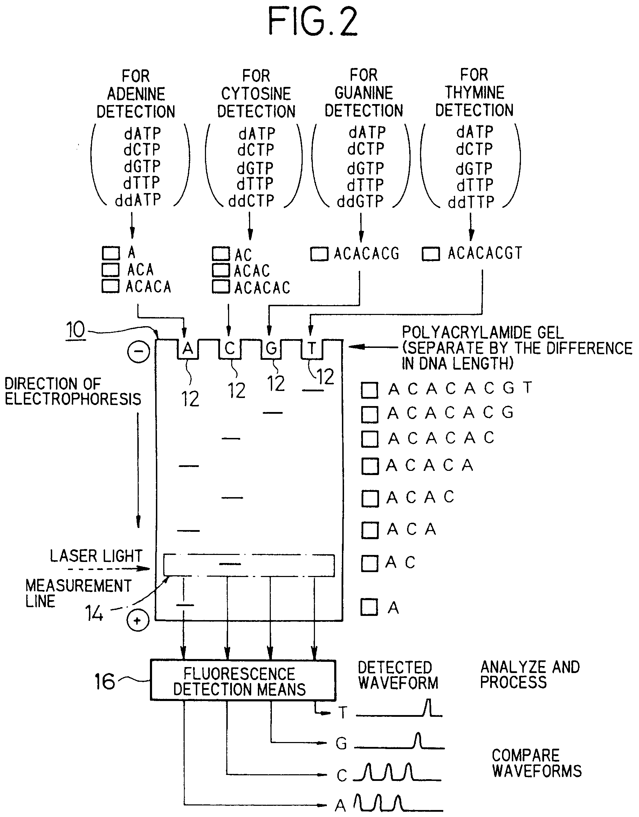

Fig. 2 shows conceptually a scheme in which the DNA fragments prepared by the sequence reaction are separated by gel electrophoresis, the separated fragments are illuminated with laser light and the fluorescence emitted from the respective fragments is received and processed to determine their base sequences. A gel electrophoretic apparatus that is to be used in implementing the method of the invention and which is generally indicated by 10 in Fig. 2 is known by being disclosed in a prior art reference such as Japanese Laid-Open Patent Application (kokai) No. 21556/1988. As is well known to one skilled in the art, gel electrophoresis is performed using an electrophoresis plate 10 having an electrolyte layer such as a polyacrylamide gel interposed between two glass plates. As taught in Japanese Laid-Open Patent Application (kokai) No. 21556/1988, supra, the electrophoresis plate 10 is held in a vertical position. The upper end of the electrolyte layer is provided with four specimen injection dents 12 which correspond to A, C, G and T. A site for launching laser light (i.e., a measurement line) 14 is located about 90 - 100 mm below the bottom of the dents. As shown in Fig. 2, the apparatus is so adapted that laser light is launched from one side of the electrophoresis plate in such a way that it traverses the electrolyte layer in a horizontal direction. Although not shown, both the upper and lower parts of the electrophoresis plate are submerged in a buffer solution, through which a voltage is applied to the electrolyte layer to separate DNA fragments in the dents 12. Since DNA is a chained polymer with negative charges, it will move across the gel at a rate in inverse proportion to its molecular weight. The shorter the DNA chain (the smaller its molecular weight), the faster will it move and vice versa; this is the principle behind the fractionation of DNA by molecular weight.

All DNA fragments contain primer regions labelled with a fluorophore for the dideoxy reaction and the fluorophore will emit weak fluorescence upon excitation with laser light. This effect, combined with polyacrylamide gel electrophoresis, has enabled DNA fragments to be separated by size and detected on a real-time basis. The fluorescence detection system relying upon this principle is used in DNA base sequencing but it has serious drawbacks in connection with the rapid detection of variations in the base sequences of DNA. The drawbacks concern primarily the method of fluorescence detection and the system of analyzing the results of detection. One of the problems is the need to employ a system for recording all information in the gel per unit time (i.e., the time required for detecting the emitted fluorescence and feeding the detection output into a computer). This results in the detection of fluorescent emission from the electrophoretic lanes for two base species in the presence of heterozygotic (mosaic) mutation. In this case, a mismatch in the detection time will cause a difference in the intensity of emitted fluorescence. If two base species that should inherently emit fluorescent beams at an intensity ratio of 1:1 produce an intensity ratio of 1:3, erroneous results will occur. Similarly, if different detectors (e.g. photodiodes) are used for individual electrophoresis lanes, the latter might spread laterally due to variations in the detectors or fluctuations in electric field during electrophoresis (this phenomenon is generally called "smiling") and the resulting departure or deviation of lanes from the associated detectors will also cause erroneous results. This problem can be solved by using a multi-channel line sensor and launching excitation light from one lateral side of the gel. A second problem concerns the sequence conversion which has been performed on the data that are obtained in the heretofore adopted base sequencing methods. Sequence conversion is necessary for determining the base sequence of an unknown DNA sample but unnecessary for detecting a variation in a given sequence or a known variation. Therefore, if PCR products amplified by using the same amplifying primers are turned into fragments with the same enzyme using the same labelling primer, the detection data from the same base species will draw peak patterns at completely identical intensity ratios. It should also be noted that when detecting information from such peak patterns, the resolution need not be as high as in the case of sequence analysis which requires all bases under analysis to be separated one by one; this offers the advantage of shortening the lengths of electrophoresis tracks, thereby enabling the accomplishment of rapid electrophoresis.

As shown in Fig. 2, four fluorophore-labelled DNA fragments that are respectively used for detecting A, C, G and T are injected into assigned dents 12 and a voltage is applied to the electrolyte layer. In the method of the invention, electrophoresis track length is comparatively short (90 - 100 mm) and, hence, an applied voltage of about 650 - 800 volts will suffice. If a higher gel concentration is used in order to further shorten the electrophoresis time, a higher voltage, say, 1000 - 1500 volts, may be applied. When the fluorophore-labelled DNA fragments migrating through the gel pass the measurement line 14, they will fluoresce successively. The emitted fluorescence is received by fluorescence detection means 16. The specific types of terminal bases can be identified from the horizontal position of the fluorescent emission. The identified base types are picked up as detection waveforms and compared with known waveforms having normal base sequences. If there is any difference between the two waveforms, one may well assume that the subject under screening has something abnormal in his genetic base sequence. Such a suspect may then be subjected to a more precise examination to determine whether the abnormal base sequence is real, where the mutation is, which type of mutation it is and the name of the possible disease. In Fig. 2, the square mark "□" put before each DNA fragment indicates a primer.

Needless to say, the apparatus shown in Fig. 2 may be used to determine the base sequences of DNA fragments. However, the method of the invention is primarily intended for locating a limited number of suspects from a large population of subjects at low cost; therefore, in principle, only comparison of waveforms is performed in the invention and no determination of base sequences is done. As for the suspects, their base sequences may be delivered as separate outputs for later use in subsequent precise examination.

The following examples are provided for the purpose of further illustrating the method of the invention but are in no way to be taken as limiting.

### Example 1

The sequence of a gene coding for human α-ketoglutaric acid dehydrogenase involved one polymorphic base substitution and the method of the invention was implemented to detect this substitution.

### Step 1: Extracting DNA

Chromosomal DNA was extracted from lymphocytes in human peripheral blood samples from four subjects and purified for use in the following steps.

### Step 2: Amplifying he target DNA region (followed by extraction and purification of the amplified product)

Using a heat-resistant enzyme Taq polymerase, the mutation containing region was amplified by PCR. As primers for amplification by PCR, two sequences were synthesized in such positions that they held the site of mutation therebetween; one sequence was complementary to 21 bases in sites 279 - 310 bases apart from the site of mutation toward the 5' end while the other sequence was complementary to 21 bases in sites 144 - 165 bases apart from the site of mutation toward the 3' end; both sequences were prepared with a Cyclone Plus DNA/RNA sequencer of Millipore, Inc.
- Primer 1:: 5'-AGGACTAGAGAATAGTGCCTT-3'
- Primer 2:: 5'-AGGCCTTTGGATACAAAGTCT-3'
The two primers (15 pM each), the chromosomal DNA (50 ng) and the Taq polymerase (4 units) were added to give a final volume of 100 µl and the mixture was subjected to amplification by PCR, which was conducted through 30 cycles. One PCR cycle consisted of:

| | |
|---|---|
| Dissociating reaction | 94°C x 60 sec |
| Pairing reaction | 56°C x 60 sec |
| Extending reaction | 72°C x 60 sec |

The PCR product obtained by the amplification reaction was a double-stranded DNA having a total length of 465 bp. This PCR product was recovered from the 100-µl reaction solution by precipitation with a surfactant polyethylene glycol (PEG) and then purified. The purified amplification product was dissolved in 20 - 30 µl of H₂O and used in the subsequent step.

### Step 3: Preparing fluorophore-labelled DNA fragments (by the sequence reaction)

The amplification product was processed into DNA fragments by a modified version of the Sanger method using the Taq polymerase and the PCR apparatus. The Sanger method is also known as the dideoxy-reaction method or the chain-termination method; in this method, deoxynucleotide triphosphates (dNTP) for four bases (adenine, A; cytosine, C; guanine, G; thymine, T) are used as substrates in the synthesis of DNA chains (chain reactions) and a dideoxynucleotide triphosphate (ddNTP) for one base is added to these substrates, so that either dNTP or ddNTP will be incorporated during synthesis at the site of that particular base species. If dNTP is incorporated, the synthesis reaction will be further extended but if ddNTP is incorporated, the synthesis reaction will stop to yield a fragment that is terminated with that particular base species. This is because a mixture of dNTP and ddNTP in certain proportions allows ddNTP to be incorporated in a suitable proportion into the DNA chain being synthesized. The incorporation occurs at all sites of the same base species that are present in the sequence being synthesized, generating DNA chains of varying sizes (lengths). Therefore, if reaction is performed on ddNTPs for the four base species, DNA chains of different sizes (lengths) will be generated for individual single bases; the DNA chains are then subjected to electrophoresis for separation by size and the base sequence of the DNA at issue can be identified by arranging the separated bases in order. Speaking of a primer for serving as the start point for the DNA chain extending reaction by PCR, the following sequence was used that was complementary to 24 bases 166 - 190 bases apart from the site of mutation toward the 5' end: 5'-GCTTGGTTGCTTCTCATTTCAGAC-3'. During its synthesis, the primer was labelled for fluorescence by attaching a fluorophore fluorescein isothiocyanate to its 5' terminal end. This labelled primer (1.8 pM), the PCR product as a template DNA (0.5 pM) and the heat-resistant enzyme Taq polymerase (4 units) or Vent polymerase (2 units) were added and the mixture was subjected to the dideoxy reaction by PCR, which was conducted through 30 cycles. One PCR cycle consisted of:

| | |
|---|---|
| Dissociating reaction | 94°C x 20 sec |
| Pairing reaction | 64°C x 15 sec |
| Extending reaction | 70°C x 60 sec |

### Step 4: Separating and detecting DNA fragments

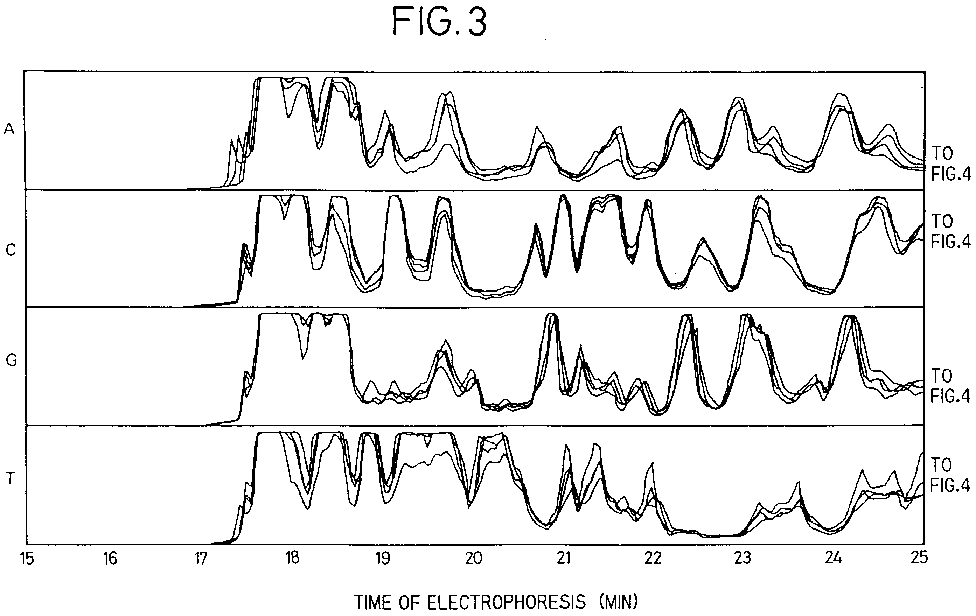

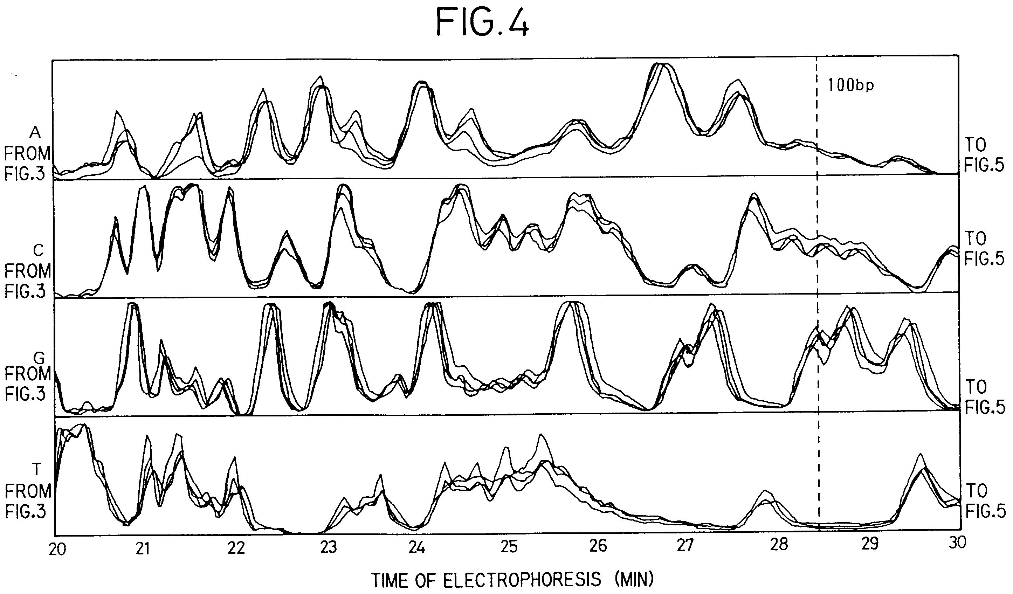

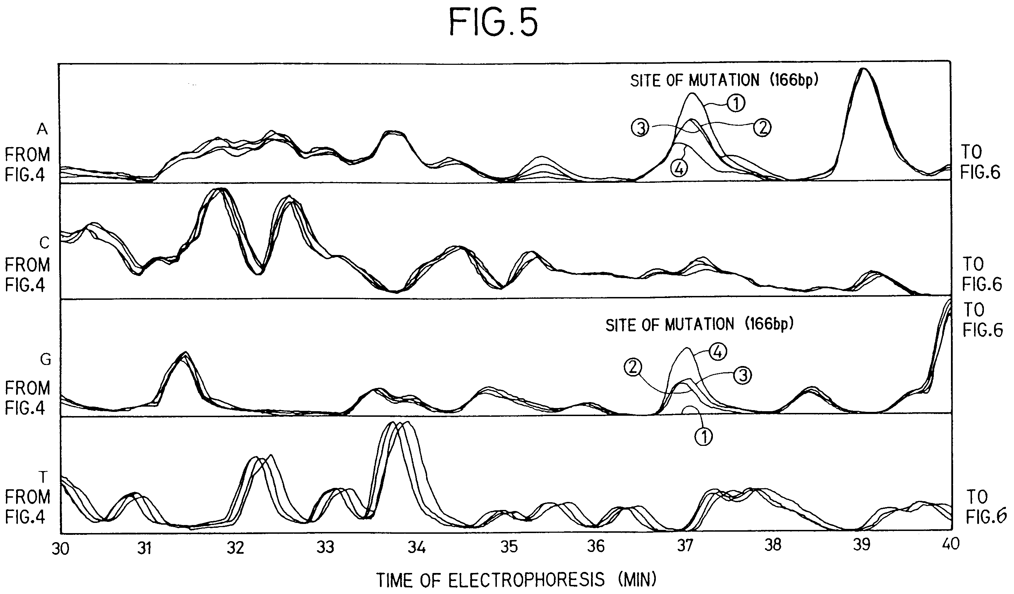

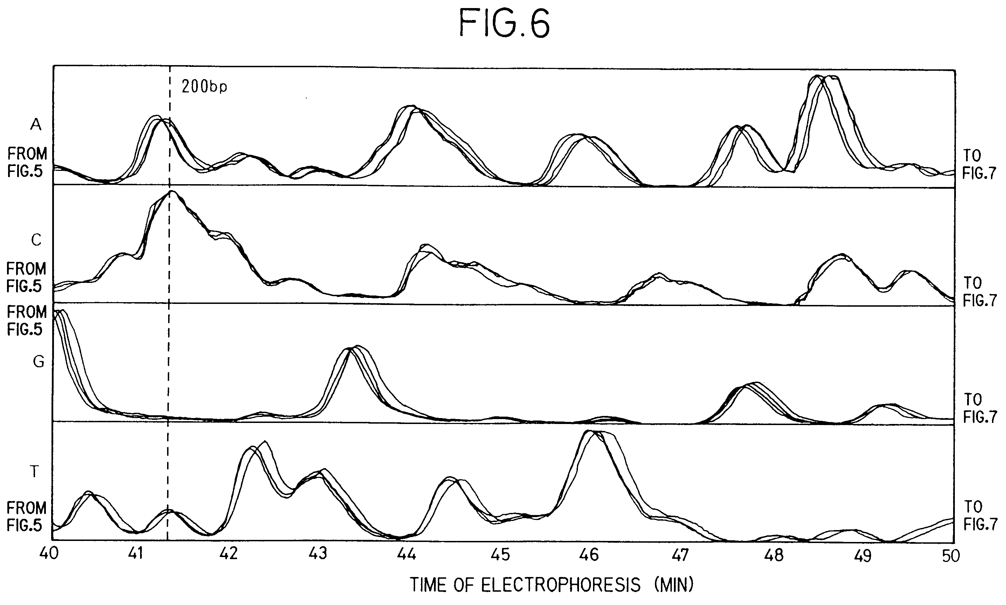

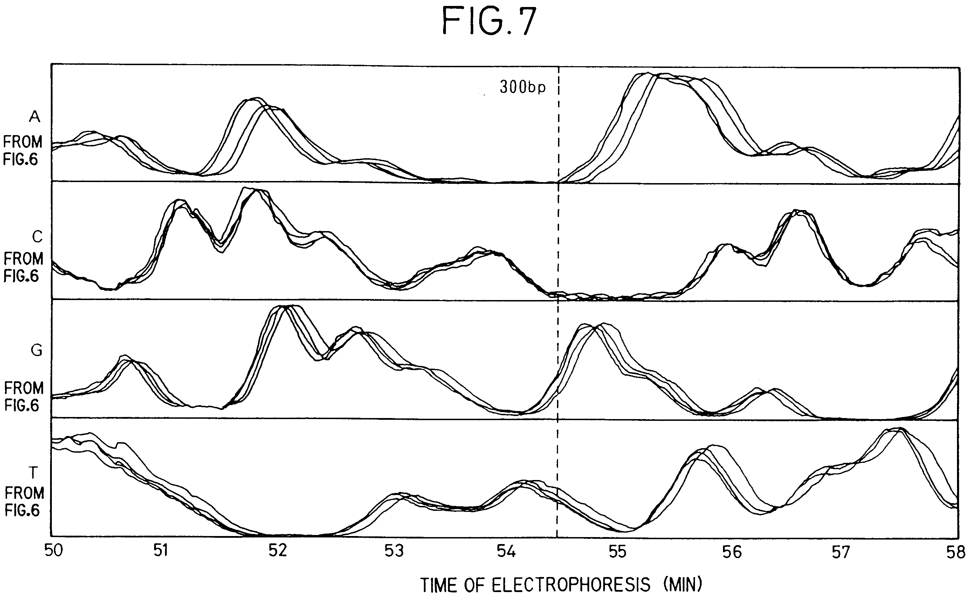

Using SQ-3000/32 Ver. 2.OOR (equipped with an argon laser) commercially available from Hitachi Electronics Engineering Co., Ltd., the fluorophore-labelled fragments prepared in step 3 were subjected to analysis by gel electrophoresis under the following conditions: fragment separating track length, 100 mm; gel, 4.5% polyacrylamide gel; applied voltage, 800 V; current, 20 mA; buffer solution, TBE buffer (liquid mixture of pH 8.0 consisting of 0.09 M Tris, 0.09 M boric acid and 0.002 M EDTA). The detected waveforms are shown in Figs. 3 - 7. As is clear from Fig. 3, the labelled primer was detected when about 17 minutes passed after the start of electrophoresis. As is clear from Fig. 4, the base in position 100 was detected after the lapse of about 28 min. As is clear from Fig. 5, the abnormal site in the base sequence that corresponded to the base in position 166 was detected when about 37 minutes passed after the sample was subjected to electrophoresis; thus, it was possible to evaluate the mutation by the differences in peak patterns. Upon closer examination, the differences in peak patterns were as follows: the proper sequence should consist of two successive A's but as it turned out, the second appearance of A was of type AA (father, A; mother, A) in peak (1), of type AG (father was either A or G and mother was either G or A) in peaks (2) and (3), and of type GG (both father and mother were G) in peak (4). In peak (4), the first appearance of A in the sequence consisting of two successive A's was detected but the second appearance of A was not. In the case described above, the subjects having essentially coinciding peaks (2) and (3) may well be judged as healthy persons but the subjects having peaks (1) and (4) that differ substantially from peaks (2) and (3) may well be regarded as suspects who need be rechecked by more precise examination. Upon continued electrophoresis, the base in position 200 was detected after the lapse of about 41 minutes as shown in Fig. 6. Further electrophoresis gave the result shown in Fig. 7, from which one can see that the base in position 300 was detected after the lapse of about 54 minutes. It is therefore understood that fluorophore-labelled DNA fragments having lengths of about 300 bp can be analyzed within about one hour by the method of the invention.

As mentioned hereinabove, the rate of electrophoresis can be varied by changing the thickness of the polyacrylamide gel, its length (separating track length), its concentration, the voltage applied to it and the formulation of the buffer solution in which the gel is to be submerged. Therefore, more rapid detection of abnormal sites in base sequences could be realized by setting optimal conditions as regards the time of data entry from the detector to a computer and rate-related variable factors.

When the primary screening method of the invention is to be used for detecting abnormal sequences in which the sites of mutation are limited, four lanes (A, C, G and T) are usually employed per sample; however, if only substituted base species (one or two species) are processed into fragments and analyzed, the method will be capable of handling two to four times as many as the samples that can be conventionally processed. If two or more samples of the same base species are mixed and detected for each lane, the throughput of the method will further increase by a factor of at least two and this possibility is suggested by the fact that both hetero and homo binding could be detected. In the case of hetero binding, this fact means that peaks were detected at a height one half the height of homozygotic peaks in the samples under electrophoresis. If one of the two mixed samples shows normal peaks whereas the other shows abnormal peaks, the result is theoretically the same as in the case of heterozygosis.

The applicability of the primary screening method of the invention is not limited to PKU and OTC deficiencies; it is also useful in detecting carriers of other diseases that are characterized by high incidence of gene mutations, such as medium-chain acyl CoA dehydrogenase deficiencies, Tay-Sachs disease, Gaucher's disease, familial hyperlipidemia, familial amyloidosis, dihydropteridine reductase deficiencies and Fabry's disease.

As described on the foregoing pages, the method of the invention insures that carriers who have abnormal base sequences in their genes can be located from a large population of subjects with great rapidity and at low cost. Since precise examination need be performed only on the carriers picked up by the method, the overall time and cost for examinations can be drastically reduced compared to the case where precise examinations have to be performed on all the subjects under screening.

Examinations at the level of gene analysis such as DNA diagnosis can be performed satisfactorily by implementing the method of the invention on four bases A, T, C and G. Even if the results of such examinations are positive, there may not be the need to perform precise examinations such as by quantification of abnormal metabolites or enzymological methods or by expression of characters. This is because the currently available data base is the results of precise examinations that have so far been conducted on patients with certain diseases by ingenious techniques such as enzymological expression of characters and which are, therefore, characterized by established correlations between mutations and diseases. However, in future searching for mutations, humans might be detected who harbor the same mutation as patients but who are apparently normal (i.e. show no manifestation of a disease). Stated more specifically, cases might in future be discovered in which the disease that is induced by a certain mutation is repressed by the mutation of a different site. Hence, collation or checking against the results of examinations other than gene analysis would be necessary before the repressed mutation or the pertinent repressor is identified. Such collation would also be necessary in cancers and other cases where mutations will not result in immediate manifestations of diseases but require aging as a complex inducing factor.

## Claims

1. In a method of primary screening for carriers having abnormal DNA base sequences that comprises the steps of amplifying a human extracted DNA by PCR, treating the PCR amplified product by a sequence technique to prepare DNA fragments, provided that the DNA is labelled with a fluorophore during the treatment by said sequence technique, then injecting the fluorophore-labelled DNA fragments into dents at the upper end of the electrolyte layer in the electrophoresis plate of a gel electrophoretic apparatus, applying a voltage to said electrolyte layer so that said DNA fragments are migrated, applying laser light to the migrating DNA fragments in a position a predetermined distance away from said dents, receiving the emitted fluorescence from said fragments by fluorescence detecting means, and outputting the reception signal as a waveform, characterized in that the fluorophore-labelled DNA fragments prepared by said sequence technique have lengths not more than 500 base pairs, that the length of electrophoresis tracks extending from the bottom of said dents to the point of incidence of laser light is no more than 100 mm, and that the base sequence of the DNA in each fraction is checked for the site of mutation by comparing said waveform output with the reference waveform of a normal subject.

2. A method according to claim 1 wherein the length of the fluorophore-labelled DNA fragments have lengths of from 100 to 400 base pairs.

3. A method according to claim 2 wherein the length of the fluorophore-labelled DNA fragments have lengths of from 100 to 300 base pairs.

4. A method according to claim 1 wherein the length of electrophoresis tracks is in a range of from 90 to 100 mm.

5. A method according to claim 1 wherein the applied voltage is in a range of from 650 to 800 volts.

6. A method according to claim 1 wherein PCR is implemented using primers for amplification by PCR, said primers being set at sites that are at least 30 base pairs distant from known sites of base substitution, and wherein said primers located at the favorable sites of sequence are 18 - 30 base pairs long and satisfy the following conditions: (1) they should not contain any specific sequences such as Alu and Ll that occur in human base sequences at intervals of several hundred base pairs; (2) the two primers should have substantially identical values of melting temperature (Tm); (3) the numbers of adenines (A), thymines (T), guanines (G) and cytosines (C) contained should be adjusted in such a way that the calculated Tm value is about 60°C; (4) the base at the 3' end of the sequence of each primer should be G or C; and (5) the eight bases at the 3' end of the sequence of each primer should contain more G and C than A and T.

7. A method according to claim 1 wherein the sequence technique uses a primer which satisfies the following conditions: (1) it should be located in a position at least 20 base pairs distant from the site of substitution in a direction that permits searching for the site of substitution and inside the primer that has been used to prepare the template DNA chains (i.e., within the amplified DNA chains); (2) it should be 20 - 25 base pairs long; (3) it should not contain any specific sequences such as Alu and Ll that occur in human base sequences at intervals of several hundred base pairs; (4) the numbers of adenines (A), thymines (T), guanines (G) and cytosines (C) contained should be adjusted in such a way that the calculated Tm value is about 70°C; and (5) it should have a fluorophore attached to the 5' terminal end.

8. A method according to claim 1 wherein an argon ion laser is used as a laser light source, wherein the gel electrolyte layer is made of a polyacrylamide gel, and the fluorophore is selected from the group consisting of fluorescein isothiocyanate (FITC), eosine isothiocyanate (EITC), tetramethyl rhodamine isothiocyanate (TMRITC), substituted rhodamine isothiocyanate (XRITC) and sulforhodamine 101 acid chloride.
